# EUROPÄISCHE PATENTSCHRIFT

(11) **EP 2 603 543 B1**
(45) Veröffentlichungstag und Bekanntmachung des Hinweises auf die Patenterteilung: **07.01.2015**
(21) Anmeldenummer: 11745952.9
(22) Anmeldetag: 08.08.2011
(51) Int. Cl.: C08G 65/00

(54) **VERFAHREN ZUR HERSTELLUNG VON POLYETHERALKOHOLEN**
PROCESS FOR PREPARING POLYETHER ALCOHOLS
PROCÉDÉ DE PRODUCTION DE POLYÉTHER-ALCOOLS

(30) Priorität: 09.08.2010 DE 102010039090
(43) Veröffentlichungstag der Anmeldung: 19.06.2013
(73) Patentinhaber: BASF SE, 67056 Ludwigshafen (DE)
(72) Erfinder: LOEFFLER, Achim, 67346 Speyer (DE); STOESSER, Michael, 67141 Neuhofen (DE); LOTH, Wolfgang, 67098 Bad Dürkheim (DE); BOEHLING, Ralf, 64653 Lorsch (DE); ZARBAKHSH, Sirus, Hong Kong (CN)
(86) Internationale Anmeldenummer: PCT/EP2011/063646
(87) Internationale Veröffentlichungsnummer: WO 2012/020005

(56) Entgegenhaltungen:
- EP-A1- 1 586 372
- WO-A1-00/74845
- WO-A1-2007/135154
- WO-A2-02/09866
- EDENS M W ET AL: "APPLICATIONS OF BLOCK COPOLYMER SURFACTANTS", DEVELOPMENTS IN BLOCK COPOLYMER SCIENCE AND TECHNOLOGY, WILEY, US, 1. Januar 2004 (2004-01-01), Seiten 326-340, XP001233807,

## Beschreibung

Die Erfindung betrifft ein Verfahren zur Herstellung von Polyetheralkoholen.

Polyetheralkohole können Polyetherpolyole sein, wobei diese insbesondere als Rohstoffe für die Herstellung von Polyurethanen eingesetzt werden, oder auch Polyethermonoole, die in unterschiedlichen Anwendungsfeldern zum Einsatz kommen, insbesondere als grenzflächenaktive Substanzen, Wasch- und Reinigungsmittel, im Mining, in der Bauchemie, als Ölfeldchemikalien, in der Textil- oder Lederbearbeitung, als Coatings, als Formulierungshilfsstoffe für Pflanzenschutzmittel, als Kosmetika und Personal Care, als Formulierungshilfsstoffe für Human- und Tierernährung, für Pigmente, für Arzneimittel oder als Kraftstoffadditive.

Die Herstellung von Polyetheralkoholen in einer Reaktionseinheit mit mehreren, parallel zueinander angeordneten Lagen, die mikrostrukturiert sind, ist bekannt.

Die EP-A 1 586 372 beschreibt einen mikrostrukturierten Reaktor und dessen Verwendung in einem Verfahren zur Herstellung von Polyetheralkoholen durch ringöffnende Anlagerung von Alkylenoxiden in Gegenwart eines festen Katalysators, wobei die chemische Prozessführung in Räumen stattfindet, die von zwei oder mehreren im Wesentlichen planparellelen Platten oder Schichten gebildet werden und wobei die Eduktvermischung einphasig flüssig in jedem Reaktionskanal einzeln erfolgt, eine Wärmetauschervorrichtung vorgesehen ist und der Reaktor bei Drücken bis zu 800 bar und Temperaturen im Bereich von 30 bis 400°C ausgelegt ist. Dadurch kann das Potential möglichst hoher Reaktionsgeschwindigkeiten durch hohe Alkylenoxiddrücke optimal ausgenutzt und Polyetheralkohole einheitlicher Qualität und mit niedrigem Gehalt an Nebenprodukten hergestellt werden.

Mikrostrukturierte Apparate sind jedoch sehr diffizile Gebilde. Bereits bei der Fertigung sind die Toleranzen so, dass speziell für Reaktionssysteme mit merklich ansteigender Viskosität über die Reaktionsdauer, wie dies der Fall bei der vorliegenden Herstellung von Polyetherpolyolen ist, führt der Druckverlust der einzelnen Kapillaren untereinander zu einer Maldistribution der Massenströme. Diese Problematik ist bei C. Amador et al. in Chem. Eng. J. 101(2004)1-3, Seiten 379-390 ausführlich dargestellt. Die Vermeidung von Maldistributionen bei Parallelschaltung von Rohrapparaten wurde bereits in den 80er Jahren versucht. Es wurden Ansätze entwickelt, welche auch bei viskositätsaufbauenden Systemen eine Gleichverteilung fördern. So wie bei der Zuführung zum mikrostrukturierten Reaktor die Druckverluste der einzelnen Kapillaren zu beachten sind, muss dieser Effekt auch bei der Nachdosierung berücksichtigt werden.

Darüber hinaus bedingt das Verfahren zur Herstellung von Polyetherpolyolen sehr hohe Drücke, die zu einer Reaktorauslegung von bis zu mehreren hundert bar zwingen.

Aus EP-A 06 114 369 ist ein verbessertes Verfahren zur Herstellung von Polyetherpolyolen in einer Reaktionseinheit mit mehreren parallel zueinander angeordneten Lagen, die mikrostrukturiert sind, bekannt, das eine verbesserte Gleichverteilung der Reaktionsmischung auf die einzelnen Reaktionskanäle gewährleistet, indem vor der Zuführung der Edukte und des Katalysators zu den Kanälen eine Verteileinrichtung und am Ende derselben eine Sammeleinrichtung für das Reaktionsgemisch vorgesehen ist.

Die EP-A 06 114 369 beschreibt ein Verfahren zur Herstellung von Polyetherpolyolen durch Umsetzung der folgenden Edukte:
a) ein oder mehrere Alkylenoxide und gegebenenfalls Kohlendioxid sowie
b) ein oder mehrere H-funktionelle Startsubstanzen,
in Gegenwart eines Katalysators, in einer Reaktionseinheit mit mehreren, parallel übereinander angeordneten Lagen A, B, die mikrostrukturiert sind, dergestalt, dass jede Lage eine Vielzahl von parallel zueinander angeordneten Kanälen aufweist, die von einer Seite der Platte bis zur gegenüberliegenden Seite derselben einen durchgehenden Strömungsweg ausbilden, wobei ein Teil der Edukte oder alle Edukte und gegebenenfalls der Katalysator bei einer Temperatur, die kleiner als die Temperatur der Umsetzung ist, in einem Mischer außerhalb der Kanäle vorvermischt und anschließend den Kanälen in den Lagen A auf einer Seite derselben zugeführt und auf der anderen Seite derselben das Reaktionsgemisch abgezogen werden und wobei durch die Kanäle von alternierend zu den Ebenen A angeordneten Ebenen B auf eine Seite derselben ein Wärmeträger zugeführt und auf der anderen Seite derselben abgezogen wird, und wobei für die Kanäle der Ebenen A an einem Ende derselben eine Verteileinrichtung für die Zuführung der Edukte und des Katalysators und am anderen Ende derselben eine Sammeleinrichtung für das Reaktionsgemisch vorgesehen ist.

Es war demgegenüber Aufgabe der Erfindung, eine weitere Verbesserung der Gleichverteilung der Massenströme in einem Verfahren zur Herstellung von Polyetheralkoholen zu gewährleisten, und dadurch weitere Verbesserungen der Ausbeuten und Selektivitäten sowie der Produkteigenschaften zu erreichen.

Die Aufgabe wird gelöst durch ein Verfahren gemäß Anspruch 1 zur Herstellung von Polyetheralkoholen durch Umsetzung der folgenden Edukte:
a) ein oder mehrere Alkylenoxide und gegebenenfalls Kohlendioxid sowie
b) ein oder mehrere H-funktionelle Startsubstanzen,
in Gegenwart eines Katalysators,
unter Ausbildung einer flüssigen Reaktionsmischung,
in einer Reaktionseinheit,
das dadurch gekennzeichnet ist, dass die Reaktionseinheit Einbauten aufweist, die eine Vielzahl von mikrostrukturierten Strömungskanälen ausbilden, die ein vielfaches Aufsplitten der flüssigen Reaktionsmischung in Teilströmungspfade und erneutes Rekombinieren derselben in geänderter Anordnung bewirken, wobei das vielfache Aufsplitten und erneute Rekombinieren vielfach wiederholt wird und wobei die mikrostrukturierten Strömungskanäle eine charakteristische Dimension, die als größtmöglicher Abstand eines beliebigen Teilchens der flüssigen Reaktionsmischung zu der dem Teilchen nächstliegenden Wand eines Strömungskanals definiert wird, im Bereich von 20 bis 10.000 µm aufweisen, und dadurch das Strömungsprofil der flüssigen Reaktionsmischung durch die mikrostrukturierten Strömungskanäle einer idealen Pfropfenströmung annähern.

Es wurde gefunden, dass eine Verbesserung der Gleichverteilung des Reaktionsgemisches über die gesamte Reaktionsfront möglich ist, indem in der Reaktionseinheit Einbauten eingesetzt werden, die mikrostrukturierte Strömungskanäle ausbilden, die als statische Mischer fungieren, und die eine wie oben definiert charakteristische Dimension aufweisen.

Hierbei handelt es sich um so genannte Split- and Recombine-Mischer, das heißt Mischer, die sich durch Stufen aus wiederkehrender Trennung und Zusammenführung von Strömen kennzeichnen.

Der vorliegend verwendete Begriff Strömungskanal oder auch Strömungspfad kann so definiert werden, dass zu einem Strömungskanal oder Strömungspfad alle Teilchen der flüssigen Reaktionsmischung zählen, die radial zur Strömungsrichtung nicht durch Einbauten oder Wände voneinander getrennt sind. Ein Strömungskanal oder Strömungspfad verbindet Mischstellen miteinander bzw. Verteiler mit Mischstellen.

Mischer, die mikrostrukturierte Strömungskanäle ausbilden werden auch als Mikromischer bezeichnet.

Wesentlich für die vorliegende Erfindung ist der Einsatz von Einbauten, die mikrostrukturierte Strömungskanäle mit einer charakteristischen Dimension im Bereich von 20 bis 10.000 µm ausbilden und dadurch das Strömungsprofil der flüssigen Reaktionsmischung von einem parabolischen Strömungsprofil weg einer idealen Pfropfenströmung annähern.

Die Herstellung der Polyalkohole im Verfahren nach der Erfindung erfolgt durch Umsetzung der folgenden Edukte:
a) ein oder mehrere Alkylenoxide und gegebenenfalls Kohlendioxid sowie
b) ein oder mehrere H-funktionelle Startsubstanzen,
in Gegenwart eines Katalysators.

Als Edukte a) können alle bekannten Alkylenoxide eingesetzt werden. Bevorzugt werden eine oder mehrere Substanzen, ausgewählt aus der nachfolgenden Aufzählung eingesetzt: Ethylenoxid, Propylenoxid, Butylenoxid, Pentenoxid, Glycidylether, Hexenoxidund/oder Styroloxid, bevorzugt Ethylenoxid, Propylenoxid, Kohlendioxid und Mischungen hiervon. Bei Butylenoxid, Pentenoxid und Hexenoxid sind alle Isomeren in reiner Form oder als Mischungen der Isomeren einsetzbar.

Kohlendioxid kann vorzugsweise in einer Menge von bis zu 25 Gew. %, bezogen auf das Gewicht des Polyetheralkohols, eingesetzt werden.

Als H-funktionelle Startsubstanz oder Startsubstanzen dienen bevorzugt ein oder mehrere Alkohole mit einer Funktionalität von 1 bis 8, bevorzugt von 2 bis 8, besonders bevorzugt von 2 bis 6, weiter bevorzugt von 2 bis 4.

Hierfür können eine oder mehrere Substanzen aus der nachfolgenden Aufzählung eingesetzt werden: Ethylenglykol, Propylenglykol, Diethylenglykol, Dipropylenglykol, Glycerin, Trimethylolpropan, Pentaerythrit, Sucrose, Saccharose, Glucose, Fructose, Mannose, Sorbitol, hydroxialkylierte (Meth)acrylsäurederivate sowie alkoxylierte Derivate der vorstehend aufgeführten H-funktionellen Startsubstanzen bis zu einem Molgewicht von etwa 1.500 D. Darüber hinaus können auch primäre und/oder sekundäre Amine sowie Thiole als Starter dienen. Möglich ist auch der Einsatz von Verbindungen, die sowohl OH als auch Allyl- oder Vinylgruppen enthalten, zum Beispiel Allylalkohol und dessen Veretherungsprodukte mit mehrwertigen Alkoholen und die in einer nachgeschalteten radikalischen Polymerisation als Ausgangsprodukte dienen können.

Als H-funktionelle Startsubstanzen werden bevorzugt ein oder mehrere Alkohole mit einer Funktionalität von 1 bis 8 eingesetzt.

Als H-funktionelle Startsubstanz oder Startsubstanzen können auch ein oder mehrere Alkohole mit einer Funktionalität von 1 mit der allgemeinen Formel R-OH eingesetzt werden, wobei R ein gesättigter oder ungesättigter Alkyl-, Aryl-, Aralkyl- oder Alkylarylrest mit 1 bis 60, vorzugsweise 1 bis 24 Kohlenstoffatomen ist, insbesondere eine oder mehrere Substanzen aus der nachfolgenden Aufzählung: Methanol, Butanol, Hexanol, Heptanol, Octanol, Decanol, Undecanol, Dodecanol oder Tridecanol, Tetradecanol, Pentadecanol, Hexadecanol, Heptadecanol, Octadecanol, Butenol, Hexenol, Heptenol, Octenol, Nonenol, Decenol, Undecenol, Vinylalkohol, Allylalkohol, Geraniol, Linalool, Citronellol, Phenol oder Nonylphenol. Als Alkylarylreste sind solche mit C₄- bis C₁₅-Alkylgruppen besonders bevorzugt.

Bevorzugt werden als H-funktionelle Startsubstanzen ein oder mehrere Alkohole mit einer Funktionalität von 2 bis 8, besonders bevorzugt von 2 bis 4, weiter bevorzugt von 2 bis 3, insbesondere eine oder mehrere Substanzen aus der nachfolgenden Aufzählung: Ethylenglykol, Propylenglykol, Glycerin, Trmethylolpropan und Pentaeritrit, eingesetzt.

Als Katalysatoren können insbesondere Multimetallcyanidkomplex-Katalysatoren oder Alkali- und Erdalkalimetallhydroxide, bevorzugt Kaliumhydroxid und Caesiumhydroxid, sowie auch andere basische Katalysatoren, wie Alkalialkoholate oder Amine, eingesetzt werden. Neben löslichen basischen Katalysatoren können auch unlösliche basische Katalysatoren, wie Magnesiumhydroxid oder Hydrotalcit verwendet werden. Weiterhin sind Brönsted-saure Katalysatoren, wie z.B. Montmorillonit oder Lewis-saure Katalysatoren, wie z.B. Bortrifluorid geeignet.

Geeignete Multimetallcyanid-Komplexkatalysatoren sind insbesondere Doppelmetallcyanidkatalysatoren (DMC), die bekannt und beispielsweise in der WO 01/083107 beschrieben sind. Sie haben zumeist die allgemeine Formel (I)

M¹ₐ[M²(CN)_{b}(A)_{c}]_{d} · fM¹gXₙ · h(H2O) · eL, (I)

wobei
M¹ ein Metallion, ausgewählt aus der Gruppe, enthaltend Zn²⁺, Fe²⁺, Co³⁺, Ni²⁺, Mn²⁺, Co²⁺, Sn²⁺, Pb²⁺, Mo⁴⁺, Mo⁶⁺, Al³⁺, V⁴⁺, V⁵⁺, Sr²⁺, W⁴⁺, W⁶⁺, Cr²⁺, Cr³⁺, Cd²⁺, Hg²⁺, Pd²⁺, Pt²⁺, V²⁺, Mg²⁺, Ca²⁺, Ba²⁺, Cu²⁺,
M² ein Metallion, ausgewählt aus der Gruppe, enthaltend Fe²⁺, Fe³⁺, Co²⁺, Co³⁺, Mn²⁺, Mn³⁺, V⁴⁺, V⁵⁺, Cr²⁺, Cr³⁺, Rh³⁺, Ru²⁺, Ir³⁺ bedeuten und M¹ und M² gleich oder verschieden sind,
A ein Anion, ausgewählt aus der Gruppe, enthaltend Halogenid, Hydroxyd, Sulfat, Carbonat, Cyanid, Thiocyanat, Isocyanat, Cyanat, Carboxylat, Oxalat oder Nitrat,
X ein Anion, ausgewählt aus der Gruppe, enthaltend Halogenid, Hydroxyd, Sulfat, Carbonat, Cyanid, Thiocyanat, Isocyanat, Cyanat, Carboxylat, Oxalat oder Nitrat,
L ein mit Wasser mischbarer Ligand, ausgewählt aus der Gruppe, enthaltend Alkohole Aldehyde, Ketone, Ether, Polyether Ester, Harnstoffe, Amide, Nitrile, Lactone, Lactame und Sulfide,
bedeuten, sowie
a, b, c, d, g und n so ausgewählt sind, daß die Elektroneutralität der Verbindung gewährleistet ist, und
e die Koordinationszahl des Liganden oder 0 bedeutet,
f eine gebrochene oder ganze Zahl größer oder gleich 0 bedeutet, sowie
h eine gebrochene oder ganze Zahl größer oder gleich 0 bedeutet.

Die Herstellung dieser Verbindungen erfolgt nach allgemein bekannten Verfahren, indem man die wässrige Lösung eines wasserlöslichen Metallsalzes mit der wässrigen Lösung einer Hexacyanometallatverbindung, insbesondere eines Salzes oder einer Säure, im folgenden auch als Eduktlösungen bezeichnet, vereinigt und gegebenenfalls dazu während oder nach der Vereinigung einen wasserlöslichen Liganden gibt. Solche Katalysatoren sowie ihre Herstellung werden beispielsweise in EP 862,947 und DE 197,42,978 beschrieben. Amorphe DMC-Katalysatoren mit hoher Aktivität sind in den folgenden Patenten beschrieben und ebenfalls einsetzbar:

Aus JP-A 4 145 123, US-A 5 470 813, EP-A 700 949, EP-A 743 093, EP-A 761 708 und WO 97/40086 sind DMC-Katalysatoren bekannt, die durch Einsatz von tert.-Butanol als organischem Komplexliganden (allein oder in Kombination mit einem Polyether (EP-A 700 949, EP-A 761 708, WO 97/40086)) den Anteil an monofunktionellen Polyethern mit endständigen Doppelbindungen bei der Herstellung von Polyetherpolyolen weiter reduzieren.

Auf die aufwendige Abtrennung der Multimetallcyanidverbindungen aus dem Polyetheralkohol nach der Herstellung kann verzichtet werden. Es ist jedoch auch möglich, eine größere Menge an Multimetallcyanidverbindungen einzusetzen und nach der Synthese des Polyetheralkohols die Menge der Multimetallcyanidverbindung im Polyol so weit abzureichern, dass der Polyetheralkohol die für die Weiterverarbeitung gewünschte Menge an Multimetallcyanidverbindungen enthält.

Die Multimetallcyanidverbindungen werden vorzugsweise in Form von Suspensionen eingesetzt, wobei die Multimetallcyanidverbindungen in organischen Verbindungen, vorzugsweise Alkoholen, suspendiert werden. Für das erfindungsgemäße Verfahren bietet es sich an, den Katalysator entweder in einem Zwischenprodukt oder im Endprodukt der Synthese zu dispergieren. Die Katalysatorsuspension sollte Konzentrationen zwischen 0,5 und 10 % aufweisen.

Die DMC-Katalysatoren sind hochaktiv. Bisher werden DMC-Katalysatoren vor allem in semi-batch Reaktoren oder kontinuierlichen rückvermischten Reaktoren (in der Regel Rührkesselreaktoren) eingesetzt. Mit diesem Reaktorkonzept wird der möglichen hohen Reaktionsgeschwindigkeit der DMC-Katalysatoren allerdings keine Rechnung getragen, vielmehr findet häufig eine Limitierung der maximalen Reaktionsgeschwindigkeiten aufgrund der begrenzten Wärmeabfuhrleistung dieser Reaktortypen statt, wobei sowohl Reaktoren mit innenliegenden Kühlschlangen als auch solche mit externen Wärmetauschern limitiert sind. Die Folge ist, dass die Reaktion bei hohen Alkylenoxiddosiergeschwindigkeiten nicht mehr bei konstanter Temperatur gefahren werden kann, was gerade bei der Herstellung von Polyetherolen für Polyurethananwendungen kritisch sein kann, da zum einen störende Geruchsstoffe aufgrund von thermischen Zersetzungsreaktionen auftreten können, zum anderen aufgrund von gebildeten höhermolekularen Nebenprodukten eine Verschlechterung der Schaumeigenschaften auftreten kann.

Die Konzentration der Katalysatoren liegt, sofern es sich um Multimetallcyanidkomplex-Katalysatoren handelt, in Abhängigkeit von den eingesetzten H-funktionellen Startsubstanzen, häufig in einem Bereich zwischen 5 und 5.000 ppm, bezogen auf das Gesamtgewicht der Einsatzstoffe.

Alkalihydroxide oder Alkalimetallalkoholate als Katalysatoren werden üblicherweise in höheren Konzentrationen, von beispielsweise 100 bis 50.000 ppm, bezogen auf das Gesamtgewicht der Einsatzstoffe, verwendet.

Werden diese basischen Katalysatoren verwendet, schließt sich an den eigentlichen Reaktionsteil eine Aufarbeitung an, bei der die Polyole neutralisiert und das Metallsalz oder -hydroxide aus dem Polyol entfernt werden. Die Neutralisation erfolgt in der Regel mit wässrigen organischen oder anorganischen Säuren wie z.B. CO2, Schwefelsäure, Phosphorsäure, Salzsäure, Milchsäure, Essigsäure oder ähnlichen Verbindungen. Die gebildeten Salze werden dann entweder ausgefällt und durch Filtration oder Zentrifugation abgetrennt oder in wässriger Phase durch Ionentauscher oder Coalescer abgetrennt. An die Abtrennung schließt sich in der Regel eine Trocknung an, bei der bei Temperaturen zwischen 80 und 160 °C bei reduziertem Druck von 5-500 mbar und bei Bedarf unter Zusatz eines Schleppgases wie Wasserdampf oder Stickstoff das Restwasser entfernt wird und das Polyol von störenden Nebenkomponenten wie Geruchsstoffen etc. befreit wird. Dieser letzte Aufarbeitungsschritt, die Entfernung von Geruchsstoffen bei vermindertem Druck und mit Hilfe eines Schleppgases, wird in der Regel auch bei Katalysatoren angewandt, die nicht aus der Reaktionsmischung entfernt werden müssen, wie z.B. DMC-Katalysatoren oder Amine, z.B. Imidazolen, Dimethylalkanolamine oder anderen Katalysatoren.

In vielen Fällen ist es vorteilhaft, zusammen mit oder anstelle der genannten Alkohole als H-funktionelle Startsubstanzen, deren Umsetzungsprodukte mit Alkylenoxiden, insbesondere mit Propylenoxid, einzusetzen, vorzugsweise Umsetzungsprodukte mit einer Molmasse bis zu 500 g/mol. Hierbei kann die Anlagerung der Alkylenoxide zur Herstellung der Umsetzungsprodukte mit beliebigen Katalysatoren erfolgen, beispielsweise mit basischen oder lewissauren Katalysatoren.

Es ist sowohl möglich, nur ein einziges Alkylenoxid als Edukt a) einzusetzen, als auch mehrere Alkylenoxide, wobei hierfür sowohl eine blockweise Anlagerung, bei der die Alkylenoxide einzeln nacheinander angelagert werden, oder eine statistische Anlagerung, bei der die Alkylenoxide gemeinsam zudosiert werden, möglich ist. Möglich sind auch Mischformen, wonach sowohl blockweise als auch statistische Abschnitte in die Polyetherkette eingebaut werden.

Die Edukte werden bevorzugt in einem Verhältnis von 1-300 Äquivalente Edukte a) (ein oder mehrere Alkylenoxide und gegebenenfalls Kohlendioxid), pro H-funktioneller Gruppe aus den Edukten b) eingesetzt.

Nach dem erfindungsgemäßen Verfahren wird bevorzugt ein Teil der Edukte oder es werden bevorzugt alle Edukte und gegebenenfalls der Katalysator zunächst außerhalb der Kanäle vorvermischt, wobei sichergestellt wird, dass die Temperatur bei der Vorvermischung kleiner ist als die Temperatur der anschließenden Umsetzung.

Bevorzugt wird als außerhalb des Reaktors angeordneter Mischer, worin ein Teil der Edukte oder alle Edukte und gegebenenfalls der Katalysator vorvermischt werden, ein mikrostrukturierte Mischer eingesetzt.

Hierfür eignen sich beispielsweise laminare Diffusionsmischer, Multilaminationsmischer, Mikromischer mit strukturierten Wänden oder Split-Recombine-Mischer.

Bei den laminaren Diffusionsmischern erfolgt die Vermischung von Teilströmen des Fluids, das an einer Mikrostruktur in eine Vielzahl mikroskopisch kleiner Strömungslamellen mit einer Dicke im Bereich von 10 bis 2.000 µm, oder auch 20 bis 1.000 µm oder auch 40 bis 500 µm aufgefächert wurde, ausschließlich durch molekulare Diffusion senkrecht zur Hauptströmungsrichtung. Eine überschlagsmäßige Auslegung des Mischers kann über die Fourier-Zahl Fo = τ/τ_{D} erfolgen. Liegt die Verweilzeit τ mindestens in der Größenordnung der Diffusionszeit τ_{D} für die transversale Vermischung, d. h. besitzt die Fourier-Zahl mindestens den Wert 1, erzielt man am Ausgang des Mischers nahezu vollständige molekulare Vermischung.

Laminare Diffusionsmischer können als einfache T- oder Y-Mischer oder als so genannte Multilaminationsmischer ausgeführt sein. Beim T- oder Y-Mischer werden die beiden zu mischenden Teilströme durch eine T- oder Y-förmige Anordnung einem Einzelkanal zugeführt. Maßgebend für den transversalen Diffusionsweg S_{Diff} ist hierbei die Kanalweite δ_{K}. Für typische Kanalweiten zwischen 100 µm und 1 mm ergeben sich für Gase sehr kleine Mischzeiten von weniger als 100 ms, wohingegen diese bei Flüssigkeiten im Minutenbereich liegen. Im Fall des Mischens von Flüssigkeiten, wie im Falle des vorliegenden Verfahrens, ist es vorteilhaft, den Mischvorgang zusätzlich, beispielsweise durch strömungsinduzierte Quervermischung, zu unterstützen.

Bei Multilaminationsmischern werden die zu vermischenden Teilströme in einem Verteiler in eine Vielzahl von Stromfäden geometrisch vereinzelt und am Austritt des Verteilers dann alternierend in Lamellen der Mischstrecke zugeführt. Bei Flüssigkeiten erreicht man mit den klassischen Multilaminationsmischern Mischzeiten im Sekundenbereich. Da dies für manche Anwendungen (z. B. bei schnellen Reaktionen) nicht ausreichend ist, wurde das Grundprinzip dahingehende weiterentwickelt, dass die Strömungslamellen nochmals zusätzlich geometrisch oder hydrodynamisch fokussiert werden. Bei der geometrischen Fokussierung geschieht dies durch eine Verengung in der Mischstrecke und bei der hydrodynamischen Fokussierung durch zwei Seitenströme, die den Hauptstrom senkrecht anströmen und so die Strömungslamellen weiter komprimieren. Durch die beschriebene Fokussierung lassen sich laterale Abmessungen der Strömungslamellen von wenigen Mikrometern realisieren, so dass selbst Flüssigkeiten innerhalb von einigen 10 ms gemischt werden können.

Bei Mikromischern mit strukturierten Wänden sind sekundäre Strukturen auf den Kanalwänden angeordnet, beispielsweise Riefen oder Stege, in einem bestimmten Winkel zur Hauptströmrichtung, bevorzugt von 45° oder 90°.

Split-Recombine-Mischer zeichnen sich durch Stufen aus wiederkehrender Trennung und Zusammenführung von Strömen aus. Bei jeder dieser Stufen wird die Lamellenzahl sukzessive verdoppelt und dadurch Lamellendicke und Diffusionsweg halbiert.

Vorteilhaft kann zunächst ein Alkylenoxid, beispielsweise Propylenoxid und der Katalysator, beispielsweise ein Multimetallcyanidkomplex-Katalysator vorgemischt und erst in einem zweiten Mischschritt die H-funktionelle Startsubstanz oder die H-funktionellen Startsubstanzen zugegeben werden.

Die Verweilzeit in der Verfahrensstufe der Vorvermischung liegt bevorzugt im Bereich von 1 bis 300 Sekunden.

Das Verfahren wird vorteilhaft kontinuierlich durchgeführt.
Gegenstand der Erfindung ist auch ein Verfahren gemäß Anspruch 1 zur Herstellung von Polyetheralkoholen durch Umsetzung der folgenden Edukte:
a) ein oder mehrere Alkylenoxide und gegebenenfalls Kohlendioxid sowie
b) ein oder mehrere H-funktionelle Startsubstanzen
in Gegenwart eines Katalysators,
in Blockfahrweise, dergestalt, dass
zwei oder mehrere Reaktionseinheiten entsprechend der vorstehenden Definition vorgesehen sind, wobei
1) die Edukte a) und b) einer ersten Reaktionseinheit zugeführt werden, unter Erhalt eines ersten Reaktionsgemisches,
2) das erste Reaktionsgemisch nach Verlassen der ersten Reaktionseinheit bevorzugt temperiert,
3) ein oder mehrere von den in der Verfahrensstufe 1) zugeführten verschiedene weitere Edukte oder die gleichen Edukte wie in der Verfahrensstufe 1) in einem von der Verfahrensstufe 1) unterschiedlichen Mischungsverhältnis unter Erhalt eines zweiten Reaktionsgemisches zugemischt werden, und das zweite Reaktionsgemisch
4) einer zweiten Reaktionseinheit zugeführt wird,
und wobei das hieraus erhaltene Reaktionsgemisch gegebenenfalls einer weiteren Reaktionseinheit zugeführt wird, wobei die Verfahrensschritte 2) und 3) entsprechend wiederholt werden.

In einer Verfahrensvariante wird als Katalysator ein Multimetallcyanidkomplex-Katalysator eingesetzt. In diesem Fall wird der Reaktionsaustrag der einzigen oder der letzten der mehreren Reaktionseinheiten einer Membrantrenneinheit zugeführt und in einen katalysatorreichen Strom (Retentat) und in einen katalysatorfreien Strom (Permeat oder Filtrat) aufgetrennt. Durch den Membranprozess ist insbesondere eine Anreicherung des Katalysators um den Faktor 2 bis 100 möglich. Das Katalysatorkonzentrat wird in den Reaktor recycliert, unter Ausschleusung kleiner Mengen, insbesondere im Bereich von 0,1 bis 3 %, um die Aufpegelung von Rückständen zu vermeiden. Das Filtrat (Produkt) wird dem Reaktionssystem entnommen.

Geeignete Membranprozesse sind die Mikro- bzw. Querstromfiltration und die Ultrafiltration. Die eingesetzten Membranen weisen Porendurchmesser im Bereich zwischen 1 nm und 1 µm und vorzugsweise zwischen 2 nm und 0,1 µm auf. Die Trennschichten der Filtermembranen können aus zum Beispiel organischen Polymeren, Keramik, Metall, Kohlenstoff oder Kombinationen daraus bestehen und müssen in dem Reaktionsmedium bei Prozesstemperatur stabil sein. Bevorzugt sind anorganische Membranen. Aus mechanischen Gründen sind die Trennschichten in der Regel auf einer ein- oder mehrschichtigen porösen Unterstruktur, die aus dem gleichen oder auch aus mindestens einem unterschiedlichen Material wie die Trennschicht besteht, aufgebracht. Beispiele sind:

| **Trennschicht** | **Unterstruktur (gröber als Trennschicht)** |
|---|---|
| Metall | Metall |
| Keramik | Metall, Keramik oder Kohlenstoff |
| Polymer | Polymer, Metall, Keramik oder Keramik auf Metall |
| Kohlenstoff | Kohlenstoff, Metall, oder Keramik |

Als Keramik können zum Beispiel α-Al₂O₃, ZrO₂, TiO₂, SiC oder gemischte keramische Werkstoffe und als Polymere zum Beispiel Polytetrafluorethylen, Polyvinylidenfluorid, Polysulfone, Polyethersulfone, Polyetheretherketone oder Polyamide eingesetzt werden.

Hierin beschrieben ist auch die Verwendung der nach dem vorstehend beschriebenen Verfahren hergestellten Polyetheralkohole zur Herstellung von Polyurethanen.

Hierin beschrieben ist auch die Verwendung der nach dem vorstehend beschriebenen Verfahren hergestellten Polyetheralkohole als: grenzflächenaktive Substanzen, Wasch- und Reinigungsmittel, Mining-Chemikalien, Ölfeldchenchemikalien, Textilhilfsmittel, Lederbearbeitungshilfsmittel, Coating-Additive, Formulierhilfsstoffe für Pflanzenschutzmittel, Hilfsstoffe für Kosmetika und Personal Care, Formulierungshilfsstoffe für Mensch- und Tierernährung, Formulierungshilfsstoffe für Pigmente, Formulierungshilfsstoffe für Arzneimittel oder Kraftstoffadditive.

Das erfindungsgemäße Verfahren zeichnet sich insbesondere durch eine weitergehende Vermeidung oder Reduzierung von Maldistributionen in den Kanälen eines mikrostrukturierten Reaktors gegenüber bekannten Verfahren aus. Entsprechend können durch das erfindungsgemäße Verfahren Verbesserungen der Ausbeuten und Selektivitäten sowie der Produkteigenschaften erreicht werden. Insbesondere bei der Verwendung von DMC-Katalysatoren und niedermolekularen, mehrfunktionellen Startern kann gegenüber bekannten Verfahren, die zu keinem oder sehr geringem Umsatz führen, mit dem erfindungsgemäßen Verfahren ein vollständiger Umsatz erzielt und der Gehalt an hochmolekularen Nebenprodukten reduziert werden. Die erhaltenen Produkte kennzeichnen sich gegenüber Produkten aus bekannten Verfahren insbesondere durch eine niedrigere Viskosität bei gleicher Molekularmasse.

In einer bevorzugten Ausführung der Erfindung wird mindestens ein Reaktor eingesetzt, der die Verweilzeitcharakteristik einer Pfropfenströmung aufweist.

Liegt in einem Rohrreaktor - oder vorliegend einem Strömungskanal - eine Pfropfenströmung ("plug-flow") vor, so kann der Zustand der Reaktionsmischung (zum Beispiel Temperatur, Zusammensetzung etc.) in Strömungsrichtung variieren, für jeden einzelnen Querschnitt senkrecht zur Fließrichtung hingegen ist der Zustand der Reaktionsmischung gleich. Damit haben alle in das Rohr eintretenden Volumenelemente die gleiche Verweilzeit im Reaktor. Bildlich gesehen durchströmt die Flüssigkeit das Rohr, als handele es sich um eine Anreihung leicht durch das Rohr gleitender Pfropfen. Zusätzlich kann die Quervermischung durch den intensivierten Stofftransport senkrecht zur Strömungsrichtung den thermischen - und Konzentrationsgradienten senkrecht zur Strömungsrichtung ausgleichen.

Trotz der meist laminaren Durchströmung lässt sich also Rückvermischung vermeiden und eine enge Verweilzeitverteilung ähnlich wie bei einem idealen Strömungsrohr erreichen.

Darüber hinaus gewährleisten die erfindungsgemäße eingesetzten Einbauten, die mikrostrukturiere Strömungskanäle ausbilden, eine hochgradige thermische Homogenität senkrecht zur Strömungsrichtung. Prinzipiell weist dabei jedes differentielle Volumenelement eine im Wesentlichen gleiche Temperatur über den jeweiligen Strömungsquerschnitt auf. Die maximal zulässigen Temperaturunterschiede in einem Strömungsquerschnitt hängen dabei von den gewünschten Produkteigenschaften ab: Bevorzugt beträgt die maximale Temperaturdifferenz in einem Strömungsquerschnitt weniger als 40°C, bevorzugt weniger als 20°C, weiter bevorzugt weniger als 10°C und ganz besonders bevorzugt weniger als 5°C.

Als Materialien für die erfindungsgemäß einzusetzenden Einbauten und Reaktionseinheiten haben sich im Bereich niedriger Temperaturen austenitische Edelstähle, wie 1.4541 bzw.1.4571, allgemein als V4A bzw. als V2A bekannt, sowie Edelstähle der US-Typen SS316 und SS317Ti, als vorteilhaft erwiesen. Bei höheren Temperaturen und unterkorrosiven Bedingungen ist PEEK (Polyetheretherketone: hochtemperaturbeständige thermoplastische Kunststoffe) ebenfalls geeignet. Es können aber auch korrosionsresistentere Hastelloy®-Typen, Glas oder Keramik als Materialien und/oder entsprechende Beschichtungen, wie beispielsweise TiN₃, Ni-TTFE, Ni-PFA oder dergleichen verwendet werden.

Bevorzugt werden Einbauten eingesetzt, die bewirken, dass das vielfache Aufsplitten der flüssigen Reaktionsmischung in Teilströmungspfade und erneute Rekombinieren 10 bis 10.000 mal wiederholt wird.

Bevorzugt werden Einbauten eingesetzt, die eine charakteristische Dimension der mikrostrukturierten Strömungskanäle in einem Bereich von 40 bis 6000 µm, bevorzugt in einem Bereich von 50 bis 4000 µm, aufweisen.

Die Erfindung ist nicht eingeschränkt bezüglich der konkreten technischen Ausführungsform der eingesetzten Einbauten, die mikrostrukturierte Strömungskanäle ausbilden. Es können alle bekannten statistischen Mischer eingesetzt werden, die die oben definierten Merkmale erfüllen, d.h. die insbesondere eine charakteristische Dimension, die als größtmöglicher Abstand eines beliebigen Teilchens der flüssigen Reaktionsmischung zu der dem Teilchen nächstliegenden Wand eines Strömungskanals definiert wird, im Bereich von 20 bis 10.000 µm, aufweisen.

Bevorzugt kann es sich hierbei um Einbauten handeln, die Reaktionsplatten sind, wobei zwei oder mehrere, parallel übereinander, in Hauptströmungsrichtung durch die Reaktoreinheit angeordnete Reaktionsplatten jeweils ein Reaktormodul bilden, wobei die Reaktionseinheit ein oder mehrere Reaktormodule aufweist, und wobei jede Reaktionsplatte eine Vielzahl von parallel zueinander, in einem Winkel α verschieden von Null zur Hauptströmungsrichtung angeordnete Schlitze mit konstanter oder veränderlicher Breite aufweist, und die unmittelbar benachbarte Reaktionsplatte eine Vielzahl von geometrisch entsprechenden Schlitzen, die in einem gleichen Winkel α, jedoch mit umgekehrten Vorzeichen, angeordnet sind, und wobei die Schlitze sämtlicher übereinander angeordneter Reaktionsplatten einen Strömungskanal ausbilden, mit einer Zuführung der Edukte in den Strömungskanal und eine Abführung der Produktmischung aus dem Strömungskanal, mit jeweils einem parallel zu den Reaktionsplatten, auf beiden Seiten des Reaktormoduls angeordneten Trennblech, das die Schlitze vollständig abschließt, sowie mit einer oder mehreren Kühl- oder Temperierplatten, die sich an jedes Trennblech, auf der dem Reaktormodul gegenüberliegenden Seite des Trennblechs, anschließt, wobei durch die einen oder mehreren Kühl- oder Temperierplatten ein Wärmeträger zirkuliert.

Reaktoren mit derartigen Einbauten werden beispielsweise von der Fa. BTS-Ehrmann unter der Bezeichnung Miprowa®-Reaktoren angeboten.

Bevorzugt beträgt der Winkel α 45°.

Weiter bevorzugt werden wie vorstehend beschriebene Einbauten eingesetzt, die zwei oder mehrere Kühl- oder Temperierplatten aufweisen, wobei jede Kühl- oder Temperierplatte eine Vielzahl von parallel zueinander, in einem Winkel α verschieden von Null zur Hauptströmungsrichtung des Wärmeträgers angeordnete Schlitze mit konstanter oder veränderlicher Breite aufweist, und die unmittelbar benachbarte Kühl- oder Temperierplatte eine Vielzahl von geometrisch gleichen Schlitzen, die in einem gleichen Winkel α, jedoch mit umgekehrten Vorzeichen, angeordnet sind aufweist, und wobei die Schlitze sämtlicher übereinander angeordneter Kühl- oder Temperierplatten einen Strömungskanal ausbilden, mit einer Zuführung des Wärmeträgers in den Strömungskanal und einer Abführung des Wärmeträgers am anderen Ende des Strömungskanals.

Die Erfindung wird im Folgenden anhand einer Zeichnung sowie von Ausführungsbeispielen näher erläutert.

In der Zeichnung zeigen im Einzelnen:
- Figur 1: die schematische Darstellung einer bevorzugten Ausführungsform von erfindungsgemäß eingesetzten Einbauten in einer Reaktionseinheit und
- Figur 2: eine schematische Darstellung einzelner Platten der in Figur 1 dargestellten Einbauten.

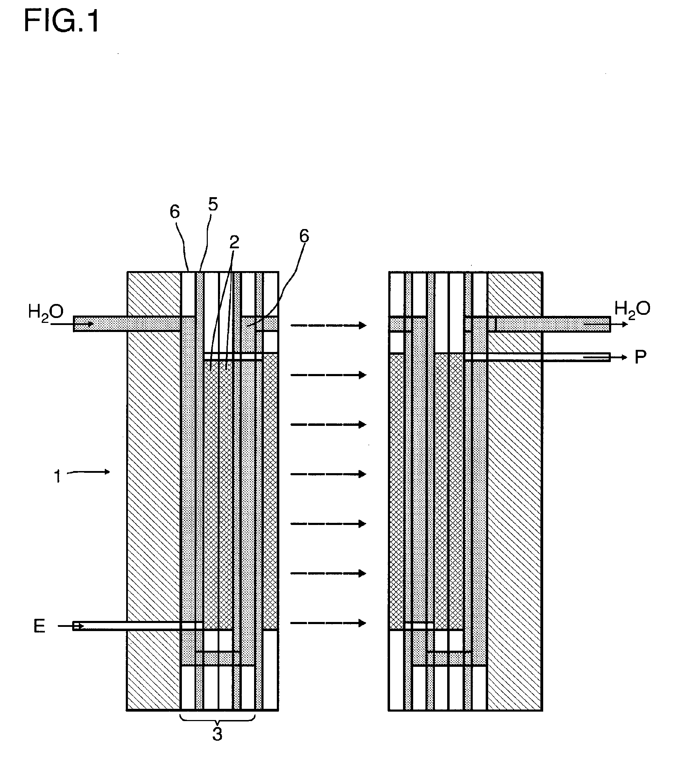

Die schematische Darstellung in Figur 1 zeigt eine Reaktionseinheit 1 mit Einbauten 2, die als zwei oder mehrere, parallel übereinander, in Hauptströmungsrichtung durch die Reaktionseinheit 1 angeordnete Reaktionsplatten 2 ausgebildet sind, und die zusammen mit an beiden Seiten derselben parallel hierzu angeordneten Trennblechen 5 sowie sich daran anschließenden Kühl- oder Temperierplatten 6 ein Reaktormodul 3 bilden. Die Edukte werden den Reaktionsplatten 2 zugeführt, durchströmen diese, und das Produkt wird am anderen Ende des letzten Reaktionsmoduls abgezogen. Kühlwasser, H₂O, durchströmt die Kühlplatten 6 im Gegenstrom zur Reaktionsmischung.

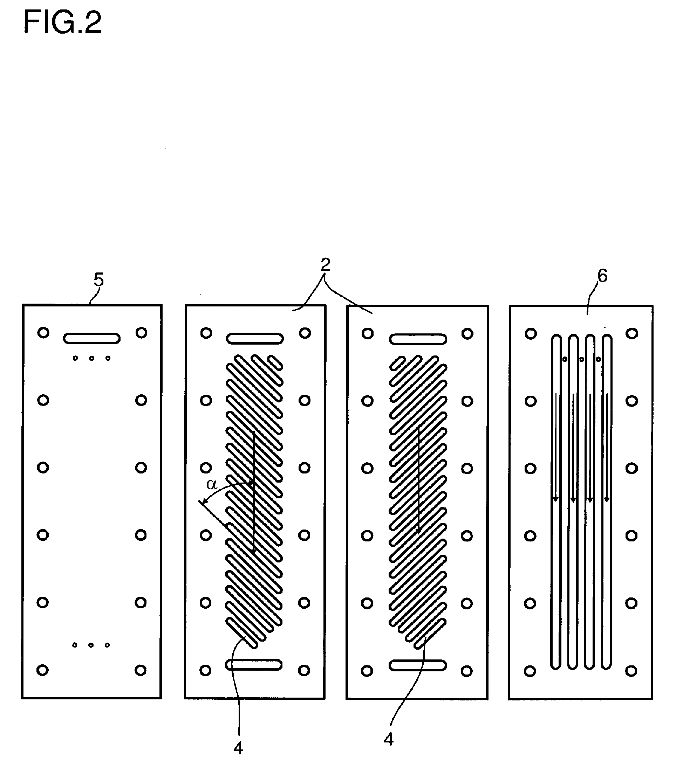

Die Darstellung in Figur 2 zeigt die einzelnen, ein Reaktormodul 3 bildenden Platten, und zwar Reaktionsplatten 2 mit Schlitzen 4, die in einem Winkel α verschieden von Null zur Hauptströmungsrichtung angeordnet sind, ein Trennblech 5 sowie eine Kühlplatte 6.

Die erfindungsgemäßen Beispiele wurden in einem Miprowa-Wärmetauscher-Reaktor der Fa. BTS Ehrmann mit den Innenmaßen 1000*12*1,5 mm durchgeführt. Der Reaktor besaß als Mischelemente drei 0,5 mm starke Kammschichten mit einer inneren Oberfläche von 180 cm².

### Ausführungsbeispiele:

### Beispiel 1: Hydroxybutylvinylether-Ethoxylate

Unter kontinuierlichem Fluss wurden das Natriumsalz eines Hyxdroxybutylvinylethers, das aus der Umsetzung des Vinylethers mit der entsprechenden Menge Natriummethanolat, gelöst in Methanol gewonnen worden war zusammen mit Ethylenoxid direkt in den oben beschrieben Mikroreaktor eingetragen.
Die Versuchsergebnisse sind der nachfolgenden Tabelle 1 zu entnehmen:

**Tabelle 1**

| | | | | | **Produkteigenschaften** | | |
|---|---|---|---|---|---|---|---|
| **Druck [bar]** | **Temp. [°C]** | **Verweilzeit [Min.]** | **EO Mol /Starter** | **Kat.-Konzentr. [%]** | **Viskosität 40°C0°C [mm²/s]** | **OH-Zahl [mg KOH/g]** | **Diol-Gehalte [%]** |
| **40** | **150** | **23** | **ca. 19** | **1,4** | **70** | **-** | **-** |

### Beispiel 2:

Unter kontinuierlichem Fluss wurden eine Natriummethanolat-Lösung, die mit einem monofunktionellen Starter, Hydroxybutylvinylether, zuvor unter Deprotonierung ins Alkoholat überführt wurde, zusammen mit Ethylenoxid direkt in den oben beschrieben Mikroreaktor eingetragen.
Die Versuchsergebnisse sind der nachfolgenden Tabelle 2 zu entnehmen:

**Tabelle 2**

| | | | | | **Produkteigenschaften** | | |
|---|---|---|---|---|---|---|---|
| **Druck [bar]** | **Temp. [°C]** | **Verweilzeit [Min.]** | **EO Mol /Starter** | **Kat.-Konzentr. [%]** | **Viskosität 40°C0°C [mm²/s]** | **OH-Zahl [mg KOH/g]** | **Diol-Gehalte [%]** |
| **40** | **150** | **23** | **ca. 27** | **1,4** | **70** | **53** | **-** |

### Beispiel 3:

Unter kontinuierlichem Fluss wurden eine Natriummethanolat-Lösung, die mit einem monofunktionellen Starter, Hydroxybutylvinylether, zuvor unter Deprotonierung ins Alkoholat überführt wurde, zusammen mit Ethylenoxid direkt in den oben beschrieben Mikroreaktor eingetragen.
Die Versuchsergebnisse sind der nachfolgenden Tabelle 3 zu entnehmen:

**Tabelle 3**

| | | | | | **Produkteigenschaften** | | |
|---|---|---|---|---|---|---|---|
| **Druck [bar]** | **Temp. [°C]** | **Verweilzeit [Min.]** | **EO Mol /Starter** | **Kat.-Konzentr. [%]** | **Viskosität 40°C0°C [mm²/s]** | **OH-Zahl [mg KOH/g]** | **Diol-Gehalte [%]** |
| **40** | **180** | **13** | **ca. 26** | **1,4** | **61** | **55** | **0,2** |

### Beispiel 4:

Unter kontinuierlichem Fluss wurden eine Natriummethanolat-Lösung, die mit einem monofunktionellen Starter, Hydroxybutylvinylether, zuvor unter Deprotonierung ins Alkoholat überführt wurde, zusammen mit Ethylenoxid direkt in den oben beschrieben Mikroreaktor eingetragen.
Die Versuchsergebnisse sind der nachfolgenden Tabelle 4 zu entnehmen:

**Tabelle 4**

| | | | | | **Produkteigenschaften** | | |
|---|---|---|---|---|---|---|---|
| **Druck [bar]** | **Temp. [°C]** | **Verweilzeit [Min.]** | **EO Mol /Starter** | **Kat.-Konzentr. [%]** | **Viskosität 40°C0°C [mm²/s]** | **OH-Zahl [mg KOH/g]** | **Diol-Gehalte [%]** |
| **40** | **180** | **8** | **ca. 25** | **1,4** | **66** | **56** | **0,1** |

### Vergleichsbeispiel 1 Polyetherpolyole für Polyurethane mit KOH-Katalyse

Unter kontinuierlichem Fluss wurde alkalischer Polyether (F=2,75, OH# = 55,6, Alkalität 0,68%, Katalysator KOH) und Ethylenoxid in einem statischen Mischer (Ehrfeld) gemischt und anschließend durch eine Kapillare ohne Einbauten mit einem Volumen von 6,1 ml (Durchmesser: 1/16") geleitet. Die Reaktionstemperatur betrug 170°C und die Umsetzung war vollständig. Die Verweilzeit in der Kapillare betrug 1,8 Minuten. Es wurde ein klares Produkt erhalten

**Tabelle 5:**

| **Druck [bar]** | **Temp. [°C]** | **Verweilzeit [Min.]** | **Mol EO /Mol Starter** | **Kat.- Konzentr. [%]** | **Viskosität 50°C [mPas]** | **OH-Zahl [mg KOH/g]** |
|---|---|---|---|---|---|---|
| | **170** | **1,8** | **ca. 20** | **0,68** | **588** | **42,3** |

### Beispiel 5 Polyetherpolyole für Polyurethane mit KOH-Katalyse

Unter kontinuierlichem Fluss wurde alkalischer Polyether (F=2,75, OH# = 55,6, Alkalität 0,67%, Katalysator KOH) und Ethylenoxid in einem statischen Mischer (Ehrfeld) gemischt und anschließend in einen Miprowa-Reaktor Fa. BTS Ehrmann mit erfindungsgemäßen Einbauten) zugeführt. Die Reaktionstemperatur betrug 170°C und die Umsetzung war vollständig. Die Verweilzeit in der Kapillare betrug 1,8 Minuten. Es wurde ein klares Produkt erhalten

**Tabelle 6:**

| **Druck [bar]** | **Temp. [°C]** | **Verweilzeit [Min.]** | **Mol EO /Mol Starter** | **Kat.-Konzentr. [%]** | **Viskosität 50°C [mPas]** | **OH-Zahl [mg KOH/g]** |
|---|---|---|---|---|---|---|
| | **170** | **1,8** | **ca. 20** | **0,67** | **432** | **42,0** |

### Beispiel 6 Polyetherpolyole für Polyurethane mit KOH-Katalyse

Unter kontinuierlichem Fluss wurde alkalischer Polyether (F=2,75, OH# = 55,6, Alkalität 0,68%, Katalysator KOH) und Ethylenoxid in einem statischen Mischer (Ehrfeld) gemischt und anschließend in einen Miprowa-Reaktor Fa. BTS Ehrmann mit erfindungsgemäßen Einbauten) zugeführt. Die Reaktionstemperatur betrug 170°C und die Umsetzung war vollständig. Die Verweilzeit in der Kapillare betrug 1,8 Minuten. Es wurde ein klares Produkt erhalten.

**Tabelle 7:**

| **Druck [bar]** | **Temp. [°C]** | **Verweilzeit [Min.]** | **Mol EO /Mol Starter** | **Kat.-Konzentr. [%]** | **Viskosität 25°C [mPas]** | **OH-Zahl [mg KOH/g]** |
|---|---|---|---|---|---|---|
| | **170** | **1,8** | **ca. 10** | **0,68** | **1441** | **47,9** |

### Beispiel 7 Polyetherpolyole für Polyurethane mit KOH-Katalyse

Unter kontinuierlichem Fluss wurde alkalischer Polyether (F=2,75, OH# = 55,6, Alkalität 0,68%) und Propylenoxid in einem statischen Mischer (Ehrfeld) gemischt und anschließend in einen Miprowa-Reaktor Fa. BTS Ehrmann mit erfindungsgemäßen Einbauten) zugeführt. Die Reaktionstemperatur betrug 170°C und die Umsetzung war vollständig. Die Verweilzeit in der Kapillare betrug 1,8 Minuten. Es wurde ein klares Produkt erhalten

**Tabelle 8:**

| **Druck [bar]** | **Temp. [°C]** | **Verweilzeit [Min.]** | **Mol PO /Mol Starter** | **Kat.- Konzentr. [%]** | **Viskosität 25°C [mPas]** | **OH-Zahl [mg KOH/g]** |
|---|---|---|---|---|---|---|
| | **150** | **1,8** | **ca. 8,7** | **0,68** | **1007** | **46,5** |

### Vergleichsbeispiel 2 Polyetherpolyole für Polyurethane mit DMC-Katalyse

Unter kontinuierlichem Fluss wurden eine DMC-Suspension in einem trifunktionellen Polypropylenoxid, Mw 3000 in einem vorgeschalteten Rührbehälter mit einem Volumen entsprechend dem 6fachen des Volumens des Mikroreaktors, vorgemischt und dann in einen Mikroreaktor (Kreuzstrom-Reaktormodul mit integriertem Zyklonmischer, Forschungszentrum Karlsruhe (FZK), Nr. 1250-X-0.0) eingetragen. Die Reaktion stoppte wiederholt und führte nur zu geringem Umsatz.

Versuchsergebnisse sind in der nachfolgenden Tabelle 9 aufgeführt:

**Tabelle 9:**

| | | | | Produkteigenschaften | | |
|---|---|---|---|---|---|---|
| **Druck [bar]** | **Temperatur [°C]** | **Verweilzeit [min]** | **Kat-Konzentration [ppm]** | **Viskosität [25°C, mPas[** | **OH-Zahl** | **PO-Konz [%]** |
| 25 | 158 | 7 | 1080 | - | - | 85 |

### Vergleichsbeispiel 3:

Der unter Vergleichsbeispiel 2 beschriebene Versuche wurde wiederholt, jedoch wurden die Edukte kontinuierlich in mikrostrukturierten Mischern vorgemischt, und zwar wurden zunächst Propylenoxid und Glycerin in einem ersten Mikromischer (Multilaminationsmischer, LH 25, Fa. Ehrfeld) und anschließend in einem zweiten, gleichartigen Mikromischer der Katalysator eingebracht und danach das Reaktionsgemisch über eine Verteilerkammer den Kanälen eines Mikroreaktor (Kreuzstrom-Reaktormodul mit integriertem Zyklonmischer, Forschungszentrum Karlsruhe (FZK), Nr. 1250-X-0.0zugeführt

Die Versuchsergebnisse sind in der nachfolgenden Tabelle 10 aufgeführt:

**Tabelle 10:**

| | | | | Produkteigenschaften | | |
|---|---|---|---|---|---|---|
| **Druck [bar]** | **Tempera tur [°C]** | **Verweil zeit [min]** | **Kat- Konzen tration [ppm]** | **Viskosität [25°C, mPas[** | **OH-Zahl** | **PO-Konz [ppm]** |
| 20 | 158 | 3 | 1050 | 2035 | 56,2 | 410 |

### Beispiel 8:

Der unter Vergleichsbeispiel 3 beschriebene Versuche wurde wiederholt, jedoch wurden die Edukte kontinuierlich in mikrostrukturierten Mischern vorgemischt, und zwar wurden zunächst Propylenoxid und Glycerin in einem ersten Mikromischer (Multilaminationsmischer, LH 25, Fa. Ehrfeld) und anschließend in einem zweiten, gleichartigen Mikromischer der Katalysator eingebracht und danach das Reaktionsgemisch einem Mikroreaktor (Miprowa-Reaktor mit erfindungsgemäßen einbauten, Fa. BTS-Ehrfeld) zugeführt
Die Versuchsergebnisse sind in der nachfolgenden Tabelle 11 aufgeführt:

**Tabelle 11:**

| | | | | | Produkteigenschaften | | |
|---|---|---|---|---|---|---|---|
| **Mischertyp** | **Druck [bar]** | **Temperatur [min]** | **Verweilzeit [min]** | **Kat-Konzen tration [ppm]** | **Viskosität bei 25°C** | **OH-Zahl** | **PO-Konz [ppm]** |
| dyn.Mischer (Rührautoklav) | 20 | 216 | 3 | 990 | 653 | 57,42 | 25 |
| dyn.Mischer (Rührautoklav) | 20 | 170 | 3 | 420 | 1085 | 53,2 | 60 |

### Beispiel 9 (mit Produkt-Rückführung):

Der unter Beispiel 2 beschriebene Versuche wurden wiederholt, jedoch wurden zunächst der DMC-Katalysator und Recyclat aus dem Mikroreaktor in einem Multilaminationsmischer (Ehrfeld, LH 25) vorgemischt und anschließend dieser Produktstrom in einem zweiten Mischer (Ehrfeld, LH 25) mit Propylenoxid und Glycerin vermischt. Das Reaktionsgemisch wurde anschließend, wie in Beispiel 8 beschrieben einem Mikroreaktor (Miprowa-Reaktor mit erfindungsgemäßen einbauten, Fa. BTS-Ehrfeld) zugeführt

Die Ergebnisse sind in der nachfolgenden Tabelle 12 aufgeführt:

**Tabelle 12:**

| | | | | | Produkteigenschaften | | |
|---|---|---|---|---|---|---|---|
| **Mischertyp** | **Druck [bar]** | **Temperatur [min]** | **Verweilzeit [min]** | **Kat-Konzen tration [ppm]** | **Viskosität bei 25°C** | **OH-Zahl** | **PO-Konz [ppm]** |
| Multilaminationsmischer | 20 | 150 | 2,7 | 2200 | 617 | 56,2 | > 1000 |

## Patentansprüche

1. Verfahren zur Herstellung von Polyetheralkoholen durch Umsetzung der folgenden Edukte:
a) ein oder mehrere Alkylenoxide und gegebenenfalls Kohlendioxid sowie
b) ein oder mehrere H-funktionelle Startsubstanzen,
in Gegenwart eines Katalysators,
unter Ausbildung einer flüssigen Reaktionsmischung,
in einer Reaktionseinheit (1),
**dadurch gekennzeichnet, dass** die Reaktionseinheit (1) Einbauten (2) aufweist, die eine Vielzahl von mikrostrukturierten Strömungskanälen ausbilden, die ein vielfaches Aufsplitten der flüssigen Reaktionsmischung in Teilströmungspfade und erneutes Rekombinieren derselben in geänderter Anordnung bewirken, wobei das vielfache Aufsplitten und erneute Rekombinieren 10 bis 10.000-mal wiederholt wird und wobei die mikrostrukturierten Strömungskanäle eine charakteristische Dimension, die als größtmöglicher Abstand eines beliebigen Teilchens der flüssigen Reaktionsmischung zu der dem Teilchen nächstliegenden Wand eines Strömungskanals definiert wird, im Bereich von 20 bis 10.000 µm aufweisen, und dadurch das Strömungsprofil der flüssigen Reaktionsmischung durch die mikrostrukturierten Strömungskanäle von einem parabolischen Strömungsprofil weg einer idealen Pfropfenströmung annähern.

2. Verfahren nach Anspruch 1, **dadurch gekennzeichnet, dass** ein Teil der Edukte oder alle Edukte und gegebenenfalls der Katalysator bei einer Temperatur, die kleiner als die Temperatur der Umsetzung ist, in einem Mischer außerhalb der Reaktionseinheit vorvermischt werden.

3. Verfahren nach Anspruch 1 oder 2, **dadurch gekennzeichnet, dass** die charakteristische Dimension der mikrostrukturierten Strömungskanäle in einem Bereich von 40 bis 6.000 µm, bevorzugt in einem Bereich von 50 bis 4.000 µm, liegt.

4. Verfahren nach einem der Ansprüche 1 bis 3, **dadurch gekennzeichnet, dass** die Einbauten (2) Reaktionsplatten (2) sind, wobei zwei oder mehrere, parallel übereinander, in Hauptströmungsrichtung durch die Reaktoreinheit (1) angeordnete Reaktionsplatten (2) jeweils ein Reaktormodul (3) bilden, wobei die Reaktionseinheit (1) ein oder mehrere Reaktormodule (3) aufweist, wobei jede Reaktionsplatte (2) eine Vielzahl von parallel zueinander, in einem Winkel α verschieden von Null zur Hauptströmungsrichtung angeordnete Schlitze (4) mit konstanter oder veränderlicher Breite aufweist, und die unmittelbar benachbarte Reaktionsplatte (2) eine Vielzahl von geometrisch entsprechenden Schlitzen (4), die in einem gleichen Winkel α, jedoch mit umgekehrten Vorzeichen, angeordnet sind, und wobei die Schlitze (4) sämtlicher übereinander angeordneter Reaktionsplatten (2) einen Strömungskanal ausbilden,
mit einer Zuführung der Edukte in den Strömungskanal und eine Abführung der Produktmischung aus dem Strömungskanal,
mit jeweils einem parallel zu den Reaktionsplatten (2), auf beiden Seiten des Reaktormoduls (3) angeordneten Trennblech (5), das die Schlitze (4) vollständig abschließt,
sowie mit einer oder mehreren Kühl- oder Temperierplatten (6), die sich an jedes Trennblech (5), auf der dem Reaktormodul (3) gegenüberliegenden Seite des Trennblechs (5), anschließt, wobei durch die einen oder mehreren Kühl- oder Temperierplatten (6) ein Wärmeträger zirkuliert.

5. Verfahren nach Anspruch 4, **dadurch gekennzeichnet, dass** der Winkel α 45° beträgt.

6. Verfahren nach Anspruch 4 oder 5, **dadurch gekennzeichnet, dass** zwei oder mehrere Kühl- oder Temperierplatten (6) vorgesehen sind, wobei jede Kühl- oder Temperierplatte (6) eine Vielzahl von parallel zueinander, in einem Winkel α verschieden von Null zur Hauptströmungsrichtung des Wärmeträgers angeordnete Schlitze (4) mit konstanter oder veränderlicher Breite aufweist, und die unmittelbar benachbarte Kühl- oder Temperierplatte (6) eine Vielzahl von geometrisch gleichen Schlitzen (4), die in einem gleichen Winkel α, jedoch mit umgekehrten Vorzeichen, angeordnet sind aufweist, und wobei die Schlitze (4) sämtlicher übereinander angeordneter Kühl- oder Temperierplatten (6) einen Strömungskanal ausbilden, mit einer Zuführung des Wärmeträgers in den Strömungskanal und einer Abführung des Wärmeträgers am anderen Ende des Strömungskanals.

7. Verfahren nach einem der Ansprüche 1 bis 6, **dadurch gekennzeichnet, dass** die Umsetzung der einen oder mehreren Alkylenoxide unter Zusatz eines oder mehrerer Comonomere aus der Klasse der zyklischen Anhydride, der Lactone, oder unter Zusatz von Kohlendioxid erfolgt.

8. Verfahren zur Herstellung von Polyetheralkoholen gemäß einem der Ansprüche 1 bis 7 durch Umsetzung der folgenden Edukte:
a) ein oder mehrere Alkylenoxide und gegebenenfalls Kohlendioxid sowie
b) ein oder mehrere H-funktionelle Startsubstanzen
in Gegenwart eines Katalysators,
in Blockfahrweise, dergestalt, dass
zwei oder mehrere Reaktionseinheiten (1) vorgesehen sind, wobei
1) die Edukte a) und b) einer ersten Reaktionseinheit zugeführt werden, unter Erhalt eines ersten Reaktionsgemisches,
2) das erste Reaktionsgemisch nach Verlassen der ersten Reaktionseinheit bevorzugt temperiert,
3) ein oder mehrere von den in der Verfahrensstufe 1 zugeführten verschiedene weitere Edukte oder die gleichen Edukte wie in der Verfahrensstufe 1) in einem von der Verfahrensstufe 1) unterschiedlichen Mischungsverhältnis unter Erhalt eines zweiten Reaktionsgemisches zugemischt werden, und das zweite Reaktionsgemisch
4) einer zweiten Reaktionseinheit zugeführt wird,
und wobei das hieraus erhaltene Reaktionsgemisch gegebenenfalls einer weiteren Reaktionseinheit zugeführt wird, wobei die Verfahrensschritte 2) und 3) entsprechend wiederholt werden.

9. Verfahren nach einem der Ansprüche 2 bis 8, **dadurch gekennzeichnet, dass** im Mischer außerhalb des Reaktors in einem ersten Mischschritt zunächst das eine oder die mehreren Alkylenoxide und gegebenenfalls Kohlendioxid mit dem Katalysator vorgemischt und in einem zweiten Mischschritt die eine oder die mehreren H-funktionellen Startsubstanzen zugemischt werden.

10. Verfahren nach Anspruch 8 oder 9, **dadurch gekennzeichnet, dass** die Edukte a) eine oder mehrere Substanzen, ausgewählt aus der nachfolgenden Aufzählung sind: Ethylenoxid, Propylenoxid, Butylenoxid, Pentenoxid, Glycidylether, Hexenoxid und/oder Styroloxid, bevorzugt Ethylenoxid, Propylenoxid, Kohlendioxid oder Mischungen hiervon.

11. Verfahren nach einem der Ansprüche 1 bis 10, **dadurch gekennzeichnet, dass** als H-funktionelle Startsubstanzen ein oder mehrere Alkohole mit einer Funktionalität von 1 bis 8 eingesetzt werden.

12. Verfahren nach Anspruch 11, **dadurch gekennzeichnet, dass** als H-funktionelle Startsubstanz oder Startsubstanzen ein oder mehrere Alkohole mit einer Funktionalität von 1 mit der allgemeinen Formel R-OH eingesetzt werden, wobei R ein gesättigter oder ungesättigter Alkyl-, Aryl-, Aralkyl- oder Alkylarylrest mit 1 bis 60, vorzugsweise 1 bis 24 Kohlenstoffatomen ist, insbesondere eine oder mehrere Substanzen aus der nachfolgenden Aufzählung: Methanol, Butanol, Hexanol, Heptanol, Octanol, Decanol, Undecanol, Dodecanol oder Tridecanol, Tetradecanol, Pentadecanol, Hexadecanoyl. Heptadecanol, Octadecanol, Butenol, Hexenol, Heptenol, Octenol, Nonenol, Decenol, Undecenol, Vinylalkohol, Allylal-kohol, Geraniol, Linalool, Citronellol, Phenol oder Nonylphenol.

13. Verfahren nach Anspruch 11, **dadurch gekennzeichnet, dass** als H-funktionelle Startsubstanzen ein oder mehrere Alkohole mit einer Funktionalität von 2 bis 8, besonders bevorzugt von 2 bis 4, weiter bevorzugt von 2 bis 3, insbesondere eine oder mehrere Substanzen aus der nachfolgenden Aufzählung: Ethylenglykol, Propylenglykol, Glycerin, Trimethylolpropan und Pentaeritrit, eingesetzt werden.

14. Verfahren nach einem der Ansprüche 1 bis 13, **dadurch gekennzeichnet, dass** als Katalysatoren Multimetallcyanidkomplex-Katalysatoren eingesetzt werden.

15. Verfahren nach einem der Ansprüche 1 bis 13, **dadurch gekennzeichnet, dass** als Katalysatoren basische Katalysatoren, wie Kaliumhydroxid, Alkalialkoholate oder Amine eingesetzt werden.

16. Verfahren nach Anspruch 14, **dadurch gekennzeichnet, dass** der Multimetallcyanidkomplex-Katalysator durch Membranquerstromfiltration des Reaktionsaustrags der einzigen oder der letzten der mehreren Reaktionseinheiten zurückgewonnen und in das Verfahren recycliert wird.

## Claims

1. A process for preparing polyether alcohols by reaction of the following starting materials:
a) one or more alkylene oxides and optionally carbon dioxide and also
b) one or more H-functional starter substances,
in the presence of a catalyst,
to form a liquid reaction mixture,
in a reaction unit (1),
wherein the reaction unit (1) has internals (2) which form a multiplicity of microstructured flow channels, which effect multiple splitting of the liquid reaction mixture into component flow pathways and renewed recombining thereof in altered arrangement, the multiple splitting and renewed recombining being repeated 10 to 10 000 times, and the microstructured flow channels having a characteristic dimension, defined as the greatest possible distance of any particle in the liquid reaction mixture to the flow channel wall closest to the particle, in the range from 20 to 10 000 µm, so that the flow profile of the liquid reaction mixture through the microstructured flow channels away from a parabolic flow profile is approximate to an ideal plug flow.

2. The process according to claim 1, wherein some of the starting materials or all of the starting materials and optionally the catalyst are premixed in a mixer outside the reaction unit at a temperature which is lower than the temperature of the reaction.

3. The process according to claim 1 or 2, wherein the characteristic dimension of the microstructured flow channels is in a range from 40 to 6000 µm, preferably in a range from 50 to 4000 µm.

4. The process according to any of claims 1 to 3, wherein the internals (2) are reaction plates (2), with two or more reaction plates (2) arranged in parallel above one another and in the principal direction of flow through the reactor unit (1) to each form one reactor module (3), and the reaction unit (1) has one or more reactor modules (3), and each reaction plate (2) has a multiplicity of slots (4) of constant or variable width which are arranged parallel to one another and at a nonzero angle α to the principal direction of flow, and the immediately adjacent reaction plate (2) has a multiplicity of geometrically corresponding slots (4) which are arranged at the same angle α but with its sign reversed, with the slots (4) of all reaction plates (2) arranged one above another forming a flow channel,
with feeding of the starting materials into the flow channel and removal of the product mixture from the flow channel,
with one separator sheet (5) arranged parallel to each of the reaction plates (2), on both sides of the reactor module (3), each separator sheet (5) completely closing off the slots (4),
and with one or more cooling or heating plates (6) adjacent to each separator sheet (5), on the side of the separator sheet (5) opposite to the reactor module (3), with a heat transfer medium circulating through the one or more cooling or heating plates (6).

5. The process according to claim 4, wherein the angle α is 45°.

6. The process according to claim 4 or 5, wherein two or more cooling or heating plates (6) are provided, with each cooling or heating plate (6) having a multiplicity of slots (4) of constant or variable width that are arranged parallel to one another and at a nonzero angle α to the principal direction of flow of the heat transfer medium, and the immediately adjacent cooling or heating plate (6) having a multiplicity of geometrically identical slots (4) which are arranged at the same angle α but with its sign reversed, and where the slots (4) of all the cooling or heating plates (6) arranged one above another form a flow channel, with the heat transfer medium being fed into the flow channel and the heat transfer medium being removed at the other end of the flow channel.

7. The process according to any of claims 1 to 6, wherein the reaction of the one or more alkylene oxides takes place with addition of one or more comonomers from the class of the cyclic anhydrides, the lactones, or with addition of carbon dioxide.

8. A process for preparing polyether alcohols according to any of claims 1 to 7 by reaction of the following starting materials:
a) one or more alkylene oxides and optionally carbon dioxide and also
b) one or more H-functional starter substances
in the presence of a catalyst,
in block operation with
two or more reaction units (1) being provided, wherein
1) the starting materials a) and b) are fed to a first reaction unit to give a first reaction mixture,
2) the first reaction mixture is preferably cooled/heated after leaving the first reaction unit,
3) one or more further starting materials different from the starting materials introduced in process step 1) or the same starting materials as in process step 1) in a mixing ratio different from that in process step 1) are mixed in to give a second reaction mixture and the second reaction mixture
4) is fed to a second reaction unit,
and the reaction mixture obtained therefrom is optionally fed to a further reaction unit, with the process steps 2) and 3) being repeated accordingly.

9. The process according to any of claims 2 to 8, wherein the one or more alkylene oxides and optionally carbon dioxide are firstly premixed with the catalyst in a first mixing step in the mixer outside the reactor and the one or more H-functional starter substances are mixed in a second mixing step.

10. The process according to claim 8 or 9, wherein the starting materials a) are one or more substances selected from the following listing: ethylene oxide, propylene oxide, butylene oxide, pentene oxide, glycidyl ether, hexene oxide and/or styrene oxide, preferably ethylene oxide, propylene oxide, carbon dioxide or mixtures thereof.

11. The process according to any of claims 1 to 10, wherein one or more alcohols having a functionality of from 1 to 8 are used as H-functional starter substances.

12. The process according to claim 11, wherein one or more alcohols having a functionality of 1 and having the general formula R-OH, where R is a saturated or unsaturated alkyl, aryl, aralkyl or alkylaryl radical having from 1 to 60, preferably from 1 to 24, carbon atoms, in particular one or more substances from the following listing: methanol, butanol, hexanol, heptanol, octanol, decanol, undecanol, dodecanol or tridecanol, tetradecanol, pentadecanol, hexadecanol, heptadecanol, octadecanol, butenol, hexenol, heptenol, octenol, nonenol, decenol, undecenol, vinyl alcohol, allyl alcohol, geraniol, linalool, citronellol, phenol or nonylphenol, are used as H-functional starter substance or substances.

13. The process according to claim 11, wherein one or more alcohols having a functionality of from 2 to 8, particularly preferably from 2 to 4, more preferably from 2 to 3, in particular one or more substances from the following listing: ethylene glycol, propylene glycol, glycerol, trimethylolpropane and pentaerythritol, are used as H-functional starter substances.

14. The process according to any of claims 1 to 13, wherein multimetal cyanide complex catalysts are used as catalysts.

15. The process according to any of claims 1 to 13, wherein basic catalysts such as potassium hydroxide, alkali metal alkoxides or amines are used as catalysts.

16. The process according to claim 14, wherein the multimetal cyanide complex catalyst is recovered by membrane crossflow filtration of the reaction discharge from the single reaction unit or the last of the multiplicity of reaction units and is recycled to the process.

## Revendications

1. Procédé de fabrication de polyéther-alcools par mise en réaction des réactifs suivants :
a) un ou plusieurs oxydes d'alkylène et éventuellement du dioxyde de carbone, et
b) une ou plusieurs substances de départ à fonction H,
en présence d'un catalyseur,
pour former un mélange réactionnel liquide,
dans une unité de réaction (1),
**caractérisé en ce que** l'unité de réaction (1) comprend des composants (2) qui forment une pluralité de canaux d'écoulement microstructurés qui effectuent une fragmentation multiple du mélange réactionnel liquide en voies d'écoulement partielles et une recombinaison de celles-ci en un ordre modifié, la fragmentation multiple et la recombinaison étant répétées 10 à 10 000 fois et les canaux d'écoulement microstructurés présentant une dimension caractéristique, définie comme la distance la plus grande possible entre une particule quelconque du mélange réactionnel liquide et la paroi d'un canal d'écoulement la plus proche de la particule, dans la plage allant de 20 à 10 000 µm, et le profil d'écoulement du mélange réactionnel dans les canaux d'écoulement microstructurés s'éloignant ainsi d'un profil d'écoulement parabolique et s'approchant d'un écoulement piston idéal.

2. Procédé selon la revendication 1, **caractérisé en ce qu'**une partie des réactifs ou tous les réactifs et éventuellement le catalyseur sont prémélangés dans un mélangeur à l'extérieur de l'unité de réaction à une température inférieure à la température de la réaction.

3. Procédé selon la revendication 1 ou 2, **caractérisé en ce que** la dimension caractéristique des canaux d'écoulement microstructurés se situe dans une plage allant de 40 à 6 000 µm, de préférence dans une plage allant de 50 à 4 000 µm.

4. Procédé selon l'une quelconque des revendications 1 à 3, **caractérisé en ce que** les composants (2) sont des plaques de réaction (2), deux plaques de réaction (2) ou plus, parallèles les unes aux autres, agencées dans la direction d'écoulement principale au travers de l'unité de réacteur (1), formant à chaque fois un module de réacteur (3), l'unité de réaction (1) comprenant un ou plusieurs modules de réacteur (3), chaque plaque de réaction (2) comprenant une pluralité de fentes (4) parallèles les unes aux autres, agencées à un angle α différent de zéro par rapport à la direction d'écoulement principale, d'une largeur constante ou variable, et la plaque de réaction (2) directement voisine comprenant une pluralité de fentes (4) de géométrie correspondante, qui sont agencées à un même angle α, mais toutefois de signe contraire, et les fentes (4) de toutes les plaques de réaction (2) agencées les unes sur les autres formant un canal d'écoulement, comprenant une alimentation des réactifs dans le canal d'écoulement et un déchargement du mélange de produits du canal d'écoulement,
comprenant à chaque fois une plaque de séparation (5) parallèle aux plaques de réaction (2), agencée des deux côtés du module de réacteur (3), qui ferme entièrement la fente (4),
et comprenant une ou plusieurs plaques de refroidissement ou de régulation de la température (6), attachées à chaque plaque de séparation (5), sur le côté de la plaque de séparation (5) opposé au module de réacteur (3), un caloporteur circulant dans la ou les plaques de refroidissement ou de régulation de la température (6).

5. Procédé selon la revendication 4, **caractérisé en ce que** l'angle α est de 45°.

6. Procédé selon la revendication 4 ou 5, **caractérisé en ce que** deux plaques de refroidissement ou de régulation de la température (6) ou plus sont prévues, chaque plaque de refroidissement ou de régulation de la température (6) comprenant une pluralité de fentes (4) parallèles les unes aux autres, agencées à un angle α différent de zéro par rapport à la direction d'écoulement principale du caloporteur, d'une largeur constante ou variable, et la plaque de refroidissement ou de régulation de la température (6) directement voisine comprenant une pluralité de fentes (4) de géométrie correspondante, qui sont agencées à un même angle α, mais toutefois de signe contraire, et les fentes (4) de toutes les plaques de refroidissement ou de régulation de la température (6) agencées les unes sur les autres formant un canal d'écoulement, comprenant une alimentation du caloporteur dans le canal d'écoulement et un déchargement du caloporteur à l'autre extrémité du canal d'écoulement.

7. Procédé selon l'une quelconque des revendications 1 à 6, **caractérisé en ce que** la réaction du ou des oxydes d'alkylène a lieu avec ajout d'un ou de plusieurs comonomères de la classe des anhydrides cycliques, des lactones ou avec ajout de dioxyde de carbone.

8. Procédé de fabrication de polyéther-alcools selon l'une quelconque des revendications 1 à 7 par mise en réaction des réactifs suivants :
a) un ou plusieurs oxydes d'alkylène et éventuellement du dioxyde de carbone, et
b) une ou plusieurs substances de départ à fonction H en présence d'un catalyseur,
en mode en bloc, de sorte que
deux unités de réaction (1) ou plus soient prévues,
1) les réactifs a) et b) étant introduits dans une première unité de réaction, pour obtenir un premier mélange réactionnel,
2) le premier mélange réactionnel étant de préférence régulé en température après avoir quitté la première unité de réaction,
3) un ou plusieurs réactifs supplémentaires différents de ceux introduits à l'étape de procédé 1 ou les mêmes réactifs que lors de l'étape de procédé 1) en un rapport de mélange différent de celui de l'étape de procédé 1) étant incorporés pour obtenir un deuxième mélange réactionnel, et le deuxième mélange réactionnel
4) étant introduit dans une deuxième unité de réaction,
et le mélange réactionnel ainsi obtenu étant éventuellement introduit dans une unité de réaction supplémentaire, les étapes de procédé 2) et 3) étant répétées de manière correspondante.

9. Procédé selon l'une quelconque des revendications 2 à 8, **caractérisé en ce que** dans le mélangeur à l'extérieur du réacteur, lors d'une première étape de mélange, le ou les oxydes d'alkylène et éventuellement du dioxyde de carbone sont tout d'abord prémélangés avec le catalyseur et, lors d'une seconde étape de mélange, la ou les substances de départ à fonction H sont incorporées.

10. Procédé selon la revendication 8 ou 9, **caractérisé en ce que** les réactifs a) sont une ou plusieurs substances choisies dans la liste suivante : oxyde d'éthylène, oxyde de propylène, oxyde de butylène, oxyde de pentène, éther glycidylique, oxyde d'hexène et/ou oxyde de styrène, de préférence oxyde d'éthylène, oxyde de propylène, dioxyde de carbone ou leurs mélanges.

11. Procédé selon l'une quelconque des revendications 1 à 10, **caractérisé en ce qu'**un ou plusieurs alcools d'une fonctionnalité de 1 à 8 sont utilisés en tant que substances de départ à fonction H.

12. Procédé selon la revendication 11, **caractérisé en ce qu'**un ou plusieurs alcools d'une fonctionnalité de 1 de formule générale R-OH sont utilisés en tant que substance de départ ou substances de départ à fonction H, R étant un radical alkyle, aryle, aralkyle ou alkylaryle saturé ou insaturé de 1 à 60, de préférence 1 à 24 atomes de carbone, notamment une ou plusieurs substances de la liste suivante : méthanol, butanol, hexanol, heptanol, octanol, décanol, undécanol, dodécanol ou tridécanol, tétradécanol, pentadécanol, hexadécanol, heptadécanol, octadécanol, buténol, hexénol, hepténol, octénol, nonénol, décénol, undécénol, alcool vinylique, alcool allylique, géraniol linalool, citronellol, phénol ou nonylphénol.

13. Procédé selon la revendication 11, **caractérisé en ce qu'**un ou plusieurs alcools d'une fonctionnalité de 2 à 8, de manière particulièrement préférée de 2 à 4, de manière davantage préférée de 2 à 3, sont utilisés en tant que substances de départ à fonction H, notamment une ou plusieurs substances de la liste suivante : éthylène glycol, propylène glycol, glycérine, triméthylolpropane et pentaérythrite.

14. Procédé selon l'une quelconque des revendications 1 à 13, **caractérisé en ce que** des catalyseurs à base d'un complexe de cyanure de plusieurs métaux sont utilisés en tant que catalyseurs.

15. Procédé selon l'une quelconque des revendications 1 à 13, **caractérisé en ce que** des catalyseurs basiques tels que l'hydroxyde de potassium, des alcoolates alcalins ou des amines sont utilisés en tant que catalyseurs.

16. Procédé selon la revendication 14, **caractérisé en ce que** le catalyseur à base d'un complexe de cyanure de plusieurs métaux est récupéré par filtration à courant transversal sur membrane de la sortie de réaction de l'unique unité de réaction ou de la dernière des unités de réaction et recyclé dans le procédé.
